# EUROPÄISCHE PATENTSCHRIFT

(11) **EP 2 483 227 B1**
(45) Veröffentlichungstag und Bekanntmachung des Hinweises auf die Patenterteilung: **01.10.2014**
(21) Anmeldenummer: 10737339.1
(22) Anmeldetag: 03.08.2010
(51) Int. Cl.: C07C 45/74, C07C 47/21

(54) **HERSTELLUNG VON ALPHA, BETA -UNGESÄTTIGTEN ALDEHYDEN MITTELS EINER REAKTIONSMISCHPUMPE**
PRODUCING ALPHA, BETA -UNSATURATED ALDEHYDES BY MEANS OF A REACTION MIXING PUMP
PRODUCTION D'ALDÉHYDES ALPHA, BETA -INSATURÉS AU MOYEN D'UNE POMPE DE MÉLANGE RÉACTIONNELLE

(30) Priorität: 30.09.2009 DE 102009045139
(43) Veröffentlichungstag der Anmeldung: 08.08.2012
(73) Patentinhaber: Evonik Degussa GmbH, 45128 Essen (DE)
(72) Erfinder: KAIZIK, Alfred, 45772 Marl (DE); LUEKEN, Hans-Gerd, 45770 Marl (DE); FRIDAG, Dirk, 45721 Haltern am See (DE); BÜSCHKEN, Wilfried, 45721 Haltern am See (DE); MACKOWIAK, Dirk, 46325 Borken (DE); BROCKSIEN, Frank, 48249 Dülmen (DE)
(86) Internationale Anmeldenummer: PCT/EP2010/061238
(87) Internationale Veröffentlichungsnummer: WO 2011/038957

(56) Entgegenhaltungen:
- EP-A1- 0 070 529
- WO-A1-2004/065342
- DE-A1- 4 220 239
- D. FINK, A. WÖLFERT: "Pumpen, die mitmischen" CHEMIE TECHNIK, 2007, Seiten 52-54, XP002599179 ISSN: 0340-9961 in der Anmeldung erwähnt

## Beschreibung

Die Erfindung betrifft ein Verfahren zur Herstellung von α,β-ungesättigten Aldehyden, bei welchem innerhalb eines kontinuierlich durchströmten Reaktors eine katalytische Aldolkondensation von Einsatzaldehyden in Gegenwart einer wässrigen Base durchgeführt wird.

Ein derartiges Verfahren ist aus WO 2004/065342 A1 bekannt.

Ungesättigte Aldehyde sind wegen ihrer Reaktivität Edukte für die Herstellung einer Vielzahl von organischen Verbindungen. Ihre Selektivhydrierung ergibt die entsprechenden gesättigten Aldehyde, die ebenfalls Basis vieler Synthesen sind. Die Oxidation der Aldehyde führt zu Carbonsäuren, die technisch genutzt werden. Die Hydrierung der Aldehyde führt zu gesättigten Alkoholen, die zur Herstellung von Weichmachern und Detergentien verwendet werden.

Die Aldolreaktion von n-Butyraldehyd unter gleichzeitiger Wasserabspaltung zu 2-Ethylhexenal wird weltweit in großem Maßstab ausgeübt, da das Hydrierprodukt, 2-Ethylhexanol, in großem Umfang als Weichmacheralkohol eingesetzt wird. Als Katalysator dient üblicherweise eine Base, gelöst in Wasser. Typisch ist die Verwendung von wässriger Natronlauge mit einem Gehalt von NaOH im Prozentbereich. Die Reaktion wird häufig in einem Temperaturbereich von 80 - 150 °C, einem Druck unter 5 bar und einem Phasenverhältnis zwischen organischer Phase und wässriger Katalysatorphase von 1 zu 20 durchgeführt (Hydrocarbon Processing, October 1980, Section 2, pages 93 - 102). Diese Reaktion kann z. B. in einem Rührkessel (DE 19 06 850, DE 927 626), in einer gepackten Säule, die im Gegenstrom betrieben wird (G. Dümbgen, D. Neubauer, Chemie-Ing.-Techn., 41, 974 (1969), oder in einem Strömungsrohr (GB 761 203) durchgeführt werden. Alle diese Verfahren liefern bei Umsätzen von 98,5 % 2-Ethylhexenal in einer Selektivität von bis zu 98 %. Nachteilig dabei ist, dass bei relativ hohen Temperaturen ein Teil des eingesetzten n-Butyraldehyds durch Cannizzaro-Reaktion irreversibel verloren geht. Die bei der Cannizzaro-Reaktion gebildete Buttersäure neutralisiert den basischen Katalysator. Es muss daher ständig ein Teil der Katalysatorlösung mit einer hohen Fracht an organischen Material ausgeschleust und durch frischen Katalysator ersetzt werden.

Analog zum n-Butyraldehyd lässt sich n-Pentanal (n-Valeraldehyd) zu ungesättigtem C₁₀-Aldehyd 2-Propyl-heptenal umsetzen. Die Aldolkondensation der C₅-Aldehyde kann in gerührten Reaktoren durchgeführt werden, die zur Abführung der Wärme mit innenliegenden Wärmetauschern ausgerüstet sind. Diese Reaktionsführung wird z. B. in WO 93/20034 A1 beschrieben und ist aufgrund der bewegten Teile mechanisch anfällig und in Bau und Instandhaltung wegen der in den Reaktor eingebauten Wärmetauscher aufwendig.

Charakteristisch für die Aldolkonaonsation von Aldehyden ist die Beteiligung am Reaktionsgeschehen von zwei flüssigen Phasen (organische Aldehyd-Phase, wässrige Katalysator-Phase), die praktisch nicht mischbar sind. Die Erzielung von hohen Umsätzen und Selektivitäten setzt daher voraus, dass die beiden ineinander nicht mischbaren Flüssigphasen während der Reaktion miteinander in innigen Kontakt gebracht werden, um den Stoffüberganghemmung zwischen den Phasen zu überwinden. Durch geeignete verfahrenstechnische Maßnahmen muss daher eine möglichst große Stoffübergangsfläche zwischen beiden Phasen erzeugt werden.

Nach dem in WO 93/20034 A1 dargestellten Stand der Technik wird der Stoffübergang zwischen der organischen Aldehyd-haltigen Phase und der wässrige Katalysatorphase bei dem Einsatz von Rührkessel durch intensives Rühren und bei der Verwendung von Rohrreaktoren durch turbulente Strömung gewährleistet.

Das Rühren im Rührkessel erfordert jedoch den Eintrag mechanischer Energie zum Antreiben der Rührorgane. Um die Produktionskosten zu senken, ist es wünschenswert, die für die Aufrechterhaltung der Reaktion erforderliche mechanische Rührarbeit möglichst effektiv einzubringen, um den Gesamtenergiebedarf zu senken.

Aus WO 2004/065342 A1 ist es bekannt, die Aldolisierung in einem Reaktor durchzuführen, welcher aus einem Rohr sowie einem Segment mit fest stehenden Verwirbelungselementen aufgebaut ist. Eine Kreiselpumpe lässt das Reaktionsgemisch durch das Rohr zirkulieren, wodurch an den Verwirbelungselementen Turbulenzen entstehen. Die turbulente Strömung der Reaktionsflüssigkeit führt zu einer Verwirbelung ihrer Phasen, wodurch der Stoffübergang zwischen ihnen ermöglicht wird. Möglicherweise handelt es sich bei der hier beschriebenen appartiven Ausführug um einen Loop-Reaktor. Vergleichsbeispiele dafür, ob die Verwirbelungseffektivität eines solchen Aldolisierungsreaktors besser ist als die eines Reaktors mit rotierenden Rührorganen liegen nicht vor.

In jedem Falle hat die in WO 2004/065342 A1 beschriebene Bauweise eines Aldolisierungsreaktors mit den außerhalb der Kreiselpumpe angeordneten, fest stehenden Verwirbelungselementen den Nachteil eines großen Eigenvolumens. Das Eigenvolumen (hold up) eines im Betrieb durchströmten Apparats beschreibt das Volumen des Fluids, welches im Ruhezustand der Anlage -bei abgeschalteten Pumpen- im Apparat verbleibt. Das Eigenvolumen des in WO 2004/065342 A1 offenbarten Aldolisierungsreaktors setzt sich aus dem Leitungsvolumen, dem Volumen des Verwirbelungssektors und des Pumpengehäuses zusammen.

Als Reaktor für die kontinuierliche Umsetzung von reaktiven und toxischen Reaktanden haben sich dank ihres geringen Eigenvolumens so genannte Reaktionsmischpumpen hervorgetan.

Eine Reaktionsmischpumpe im hier gebrauchten Sinne ist umfasst:
a) ein fest stehendes Pumpengehäuse,
b) ein im Pumpengehäuse drehbar gelagertes Laufrad, welches eine Vielzahl sich radial ersteckende, zum Umfang des Laufrad hin öffnende Mischkammern aufweist,
c) ein die Mischkammern umschließender Mischkanal, welcher sich innerhalb des Pumpengehäuse entlang des Umfanges des Laufrades erstreckt,
d) mindestens ein Zulauf für Reaktionsedukte und ein Ablauf für Reaktionsprodukte, wobei Zulauf und Ablauf über den Mischkanal fluidisch miteinander verbunden sind,
e) ein Motor, mittels welchem das Laufrad rotierend antreibbar ist.

Über die Durchführung von Reaktionen mit Hilfe von Reaktionsmischpumpen wurde bisher wie folgt berichtet:
- Phosgenumsetzung in Fink, Dieter und Wölfert, Andreas: Pumpen, die mitmischen. Chemie Technik, Juli 2007, Seiten 52 - 54.
- Polycarbonatherstellung in DE102008008841A1.
- Umsetzung von Pseudojononen zu den entsprechenden Jononen in WO 97/43254. Dabei handelt es sich jedoch um eine Cyclisierung unter sauren Bedingungen, nicht um eine Aldolkondensation unter alkalischen Bedingungen.

Bisher ist es allerdings nicht bekannt geworden, Aldoliserungen mit Hilfe von Reaktionsmischpumpen durchzuführen.

In Hinblick auf diesen Stand der Technik liegt der Erfindung die Aufgabe zu Grunde, ein Verfahren zur Aldoliserung von Aldehyden anzugeben, welches sich besonders wirtschaftlich betreiben lässt.

Gelöst wird die Aufgabe dadurch, dass als Reaktor eine Reaktionsmischpumpe verwendet wird.

Gegenstand der Erfindung ist mithin ein Verfahren zur Herstellung von α, β-ungesättigten Aldehyden, bei welchem innerhalb eines kontinuierlich durchströmten Reaktors eine katalytische Aldolkondensation von Einsatzaldehyden in Gegenwart einer wässrigen Base durchgeführt wird, wobei es sich bei dem Reaktor um eine Reaktionsmischpumpe handelt. Die Pumpe dient dabei nicht nur als Fördermittel für Lauge, Aldehyd und Produkt, sondern zugleich als Reaktor.

Die Durchführung einer Aldolkonensation mit Hilfe einer Reaktionsmischpumpe übersteigt die Erwartungen an die Wirtschaftlichkeit des Prozesses in überraschendem Maße. Dies ist wohl darauf zurückzuführen, dass die Reaktionsmischpumpe gleich auf drei Faktoren der Wirtschaftlichkeit des Prozesses positiv Einfluss nimmt: Hier ist zunächst das vergleichsweise geringe Eigenvolumen einer Reaktionsmischpumpe zu nennen, welches den toten Anteil des investierten Kapitals reduziert. Des Weiteren ist der spezifische Bedarf an mechanischer Energie der Reaktionsmischpumpe gering, da sie eine effektive Verwirbelung erzielt. Überraschenderweise kommt schließlich eine verbesserte Selektivität zum Tragen, also eine bessere Ausbeute der zu produzierenden α, β-ungesättigten Aldehyden unter geringer Bildung unerwünschter Nebenprodukte. Insbesondere der letztgenannte Effekt war nicht absehbar.

Insgesamt liegen die Vorteile des erfindungsgemäßen Verfahren in der hohen Ausbeute an α,β-ungesättigten Aldehyden im geraden Durchgang und im geringen Platzbedarf der Teilanlage für die Herstellung des Gemisches aus rohen α,β-ungesättigte Aldehyden (Gemisch vor Destillation) und im geringeren Kapitaleinsatz im Vergleich mit einer Anlage, die einen konventionellen Reaktor aufweist.

Vorteilhafte Weiterbildungen der Erfindung sind in den Unteransprüchen niedergelegt und ergeben sich aus der nun folgenden Beschreibung sowie aus den Beispielen.

### Einsatzstoffe

Das erfindungsgemäße Verfahren ist für die Umsetzung von Aldehyden oder Aldehydgemischen geeignet, die Kondensationsreaktionen eingehen können. Wird nur ein Aldehyd eingesetzt, so muss dieser zwei α-Wasserstoffatome am gleichen Kohlenstoffatom in Nachbarschaft zur CO-Gruppe besitzen. Werden zwei oder mehrere unterschiedliche Aldehyde eingesetzt, so muss mindestens einer der Aldehyde zwei α-Wasserstoffatome am gleichen Kohlenstoffatom haben.

Aldehyde mit zwei α-Wasserstoffatomen gemäß obiger Definition sind beispielsweise: Acetaldehyd, Propanal, n-Butyraldehyd, n-Valeraldehyd, 3-Methylbutyraldehyd, n-Hexanal, 3-Methylpentanal, 4-Methylpentanal, n-Heptanal, n-Octanal, n-Nonanal, n-Decanal. Diese eignen sich auch für eine Homokondensation.

Beispiele für Aldehyde mit einem α-Wasserstoffatom nach obiger Definition sind: Isobutyraldehyd, 2-Methylbutyraldehyd, 2-Methylpentanal, 2-Ethylhexanal, Cyclohexylaldehyd. Beispiele für Aldehyde mit keinem α-Wasserstoffatom sind: Benzaldehyd, 2,2-Dimethylpropanal und Formaldehyd. Die Aldehyde der beiden zuletzt genannten Gruppen können nur mit einem Aldehyd mit zwei α-Wasserstoffatomen eine Aldolkondensation eingehen.

Bevorzugte Einsatzaldehyde für das erfindungsgemäße Verfahren sind: n-Butyraldehyd, n-Valeraldehyd, ein Gemisch aus n-Butyraldehyd und Isobutyraldehyd, Gemische aus n-Valeraldehyd mit 2-Methylbutyraldehyd oder 3-Methylbutyraldehyd oder das entsprechende Dreikomponentengemisch. Insbesondere werden C₅-Aldehydgemische eingesetzt, die mehr als 90 Massen-% n-Pentanal, ganz besonders mehr als 95 Massen-% n-Pentanal enthalten. Ebenfalls kann ein Gemisch aus C₄- und C₅-Aldehyden eingesetzt werden. Diese Aldehyde können z. B. durch Hydroformylierung von Olefinen hergestellt werden.

Bei Einsatz von mehr als einem Aldehyd oder einem Aldehydgemisch können die Einzelkomponenten getrennt, vorzugsweise in der Pumpe, in den Strom der Katalysatorlösung eingespeist werden. Ebenfalls ist es möglich, alle Edukte vor der Einspeisung zu mischen und gemeinsam einzuspeisen. Weiterhin können die Aldehyde als Lösung eingesetzt werden. Als Lösungsmittel können inerte, in der Katalysatorlösung kaum lösliche Flüssigkeiten wie z. B. Kohlenwasserstoffe (Pentan, Cyclohexan, Toluol) verwendet werden.

Als Katalysator im erfindungsgemäßen Verfahren können wasserlösliche, basische Verbindungen wie z. B. Hydroxide, Hydogencarbonate, Carbonate, Carboxylate oder ihre Gemische in Form ihrer Alkali- oder Erdalkaliverbindungen verwendet werden. Vorzugsweise kommen Alkalihydroxide wie Natriumhydroxid zum Einsatz.

Die Konzentration des Katalysators in der kontinuierlichen Phase liegt zwischen 0,1 und 15 Massen-%, insbesondere zwischen 0,3 und 5 Massen-%.

Werden nach dem erfindungsgemäßen Verfahren Pentanale zu Decenalen umgesetzt, wird vorzugsweise Natronlauge als Katalysator verwendet. Ein kleiner Teil der Natronlauge wird mit dem ausgeschleusten Reaktionswasser ausgetragen. Um die Verluste an Natronlauge auszugleichen, wird frische Natronlauge zudosiert. Dabei bildet die frische Natronlauge zusammen mit der Rücklauge die Prozesslauge, die in die Reaktionsmischpumpe eingespeist wird. Die Rücklauge enthält neben Natriumhydroxid Natriumsalze von Carbonsäuren, hauptsächlich von Pentansäuren. Die Carbonsäuresalze sind im Wesentlichen durch Cannizzaro-Reaktion entstanden.

Im erfindungsgemäßen Verfahren liegt bei der Herstellung von Decenalen der Natriumgehalt der Prozesslauge am Reaktoreingang bei 0,60 bis 1,75 Massen-%, insbesondere bei 1,1 bis 1,20 Massen-%. Zur Einstellung der Natriumkonzentration der Prozesslauge wird in die Rücklauge frische Natronlauge mit einer Konzentration größer als 2,5 Massen-% eingespeist. Um wenig Wasser in das Reaktionssystem einzubringen, wird bevorzugt Natronlauge mit höherer Konzentration verwendet. Im erfindungsgemäßen Verfahren wird bevorzugt Natronlauge im Konzentrationsbereich von 5 bis 30 Massen-%, beispielsweise eine mit 10 Massen-% verwendet.

### Reaktionsmischpumpe

Für die Förderung von Edukt(en), Produkten und Katalysatorlösung sowie für die Durchführung der Umsetzung wird bei dem erfindungsgemäßen Verfahren zur Herstellung von ungesättigten Aldehyden durch Aldolkondensation eine Reaktionsmischpumpe eingesetzt. Die Reaktionsmischpumpe ist im weitesten Sinne eine Peripheralradpumpe mit mehreren Eintragsstutzen (Förderkanälen) für Edukte und Katalysatorlösung in der Umfangswand der Mischkammer der Pumpe sowie einem Austragsstutzen für das Reaktionsgemisch. Diese Apparaturen besitzen die Eigenschaften einer Pumpe, eines effektiven Mischers und die eines Reaktors. Einige dieser Apparate sind mit Kühl- bzw. Heizeinrichtungen versehen oder sie können auf anderer Weise thermostatisiert werden, sodass Reaktionen bei gewünschten Temperaturen in ihnen durchgeführt werden können.

Der Aufbau und das Funktionsprinzip der Reaktionsmischpumpen sind in der Fachliteratur (D. Fink, A. Wölfert, Chemie Technik, Juli 2007, Seiten 52 - 54) beschrieben.

Die typische Reaktionsmischpumpe (Reaktionsmischer) besteht im Wesentlichen aus dem Antrieb, dem Pumpengehäuse und dem darin befindlichen Mischrotors (Laufrad). Das Laufrad wird vom Motor vorzugsweise über eine Magnetkupplung angetrieben. Die radial auf beiden Seiten des Laufrades angeordneten Kammern (Förderzellen), der jeweilige Eintragsstutzen (Förderkanal) und ein Unterbrechsteg führen zu dem typischen Förder- und Mischverhalten von Reaktionsmischpumpen. Die Förderzellen an dem Mischrotor bilden mit den ringförmigen Kanälen an den Stirnseiten der Mischkammer die peripheralradtypischen Druckzellen. Die ausgeprägte Turbulenz im Förder- und Reaktionsraum führt in wechselseitiger Folge zum ständigen Austausch des schneller umlaufenden Flüssigkeitsinhalts der Druckzellen mit dem langsamer fließenden Flüssigkeitsstrom im Bereich der Förderkanäle und somit zu intensiven Durchmischung der Flüssigkeiten. Durch die Durchmischung wird ein homogenes und stabiles Gemisch erzeugt, welches kontinuierlich durch die Auslauföffnung ausgetragen wird. Dieser ausgeprägte Impulsaustausch und Mischeffekt der Reaktionspumpe wird in der vorliegenden Erfindung für die Durchführung der Aldolkondensation von Aldehyden angewandt.

Wie am Beispiel des erfindungsgemäßen Verfahrens gezeigt wird, ist der Einsatz von Reaktionspumpen vor allem dann bevorzugen, wenn chemisch stark reaktionsfähige Komponenten für die Umsetzung gemischt und homogen verteilt werden sollen. In Abhängigkeit von den Reaktionsbedingungen und verfahrenstechnischen Randbedingungen können die Reaktionsmischer mit speziellen Sondervorrichtungen ausgestattet werden. So können z. B. in Abhängigkeit von der Viskosität und Reaktivität der Edukte oder in Abhängigkeit der Reaktionszeit die Pumpenköpfe mit zusätzlichen Verweilzeitkammer und Vormischkammer oder mit zusätzlichen Eintragstutzen für die Rückführung von Zwischenprodukten in den Förderraum ausgerüstet werden.

### Reaktionsbedingungen

Erfindungsgemäß wird die Aldolkondensation im Temperaturbereich von 50 bis 160 °C durchgeführt. Werden Pentanale zu Decenalen umgesetzt, liegt die Reaktionstemperatur im Bereich von 100 bis 150 °C, insbesondere im Bereich von 110 bis 140 °C, ganz besonders im Bereich von 120 bis 140 °C.

Der Reaktionsdruck in der Reaktionsmischpumpe liegt mindestens so hoch, dass sowohl die Prozesslauge als auch die organischen Stoffe (Edukt, Produkt und gegebenenfalls Lösemittel) jeweils als flüssige Phase vorliegen. Bei Umsetzung von Pentanalen zu Decenalen beträgt der Druck in der Reaktionsmischpumpe 0,1 bis 2 MPa, insbesondere 0,3 bis 1 MPA, ganz besonders 0,3 bis 0,5 MPa.

Das Mengenverhältnis [kg/kg] von Prozesslauge zu Edukt am Pumpeneingang liegt im Bereich von 5 bis 500, insbesondere im Bereich von 20 bis 400. Bei der Umsetzung von Pentanalen zu Decenalen beträgt dieses Verhältnis 10 bis 300, insbesondere 40 bis 240.

Im erfindungsgemäßen Verfahren liegt die durchschnittliche Verweilzeit der Flüssigkeit (organische und wässrige Phase) in der Reaktionsmischpumpe, unter der Annahme, dass beide Phasen gleich schnell strömen, im Bereich von 0,05 bis 3 Sekunden, insbesondere im Bereich von 0,1 bis 2 Sekunden. Bei der Umsetzung von Pentanalen zu Decenalen beträgt die durchschnittliche Verweilzeit vorzugsweise 0,1 bis 1,5 Sekunden, insbesondere 0,1 bis 1 Sekunden, ganz besonders 0,2 bis 0,5 Sekunden.

### Aufarbeitung

Der Pumpenaustrag wird gekühlt und die organische Phase von der Laugenphase getrennt. Die Phasentrennung wird im Temperaturbereich von 20 bis 130 °C durchgeführt.

Bei der Abtrennung eines Decenalgemisches, entstanden aus Pentanalen, erfolgt die Phasentrennung im Temperaturbereich von 60 bis 130 °C, insbesondere im Bereich von 70 bis 120 °C, ganz besonders im Bereich von 90 bis 110 °C.

Zur Abtrennung der schweren, wässrigen Phase von der leichten, organischen Phase können Abscheider eingesetzt werden, die die Phasentrennung mit alleiniger Ausnutzung der Schwerkraft ermöglichen. Diese sogenannten Schwerkraftabscheider können auch mit Einbauten als koaleszenzfördernde Maßnahme zur Erhöhung der Trennleistung ausgeführt werden. Durch die Verwendung von Einbauten wird der Koaleszenz- und Sedimentationsprozess beschleunigt. Als Koaleszenzhilfen können z. B. Platten, Füllkörper, Gewebepackungen oder Faserbettabscheidern eingesetzt werden. Schwerkraftabscheider können als liegende Behälter oder als stehende Behälter ausgeführt werden.

Alternativ zu Schwerkraftabscheider können zur Flüssig-Flüssig-Trennung aus Separatoren nach dem Prinzip der Zentrifugen eingesetzt werden. Durch Fliehkräfte in einer sich drehenden Trommel wird dabei die schwere Phase getrennt.

Um die schwere, wässrige Phase abzutrennen, werden im erfindungsgemäßen Verfahren vorzugsweise Schwerkraftabscheider eingesetzt, bevorzugt Schwerkraftabscheider, ausgeführt als liegende Behälter mit Einbauten.

Ein Teil der abgetrennten Laugenphase wird zur Abtrennung des Reaktionswassers ausgeschleust, der andere Teil wird in den Reaktor zurückgeführt. Mit dem Ausschleusestrom werden auch ein Teil der als Nebenprodukte gebildeten Carbonsäuren (als Natriumsalze) und Natriumhydroxid abgetrennt. Dieser Strom kann einer Kläranlage zugeführt werden. Es ist jedoch auch möglich diesen Strom aufzuarbeiten und teilweise in den Prozess zurückzuführen, wie beispielsweise in DE 198 49 922 und DE 198 49 924 beschrieben.

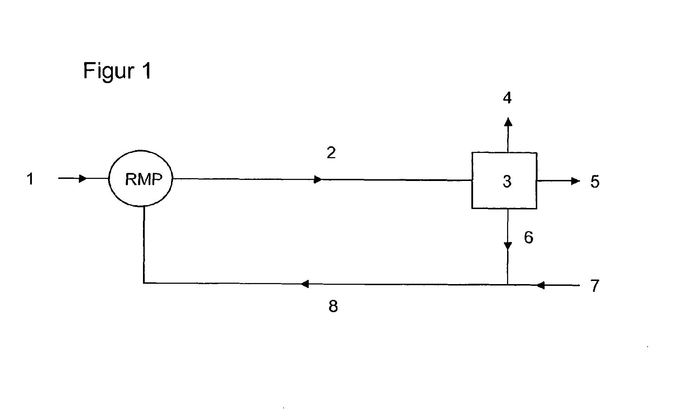

Enthält die organische Phase neben den Aldolkondensationsprodukten und geringen Mengen an nicht umgesetztem Edukt andere Nebenprodukten, wie Carbonsäuresalze, Natriumhydroxid und gelöstes Wasser, so können Basenspuren und ein Teil der Carbonsäuresalze durch eine Wasserwäsche entfernt werden. Der dabei anfallende Wasserextrakt kann zum Ansetzen der Frischlauge verwendet werden (in den Figuren 1 bis 2 nicht eingezeichnet).

Die organische Phase kann destillativ aufgearbeitet werden. Dabei abgetrennte Edukte können teilweise in die Reaktionsmischpumpe zurückgeführt werden.

Die hergestellten α,β-ungesättigten Aldehyde können zur Herstellung von Carbonsäuren (durch Hydrierung der olefinischen Doppelbindung und Oxidation der Aldehydgruppe) oder zur Herstellung von primären Alkoholen (durch Totalhydrierung) verwendet werden.

Bei der Herstellung von primären Alkoholen kann optional auch das Rohgemisch hydriert werden und die destillative Auftrennung nach der Hydrierung erfolgen.

Eine weitere Option der vorliegenden Erfindung ist, das Reaktionsgemisch nach Verlassen des Reaktors und vor der Phasentrennung einer Kurzdestillation zu unterwerfen. Dabei wird das heiße Reaktionsgemisch in einen Behälter entspannt.

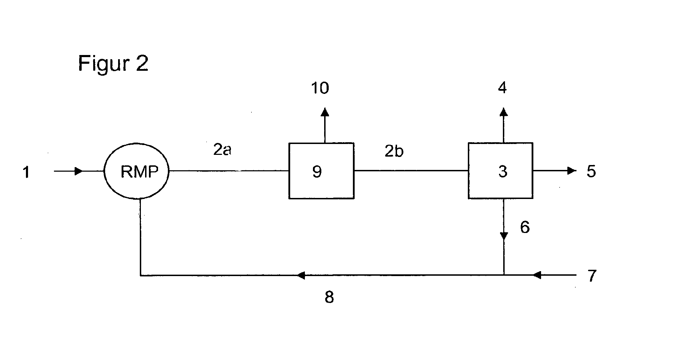

Als Destillat fällt ein Gemisch aus Wasser und hauptsächlich Edukt an, das ganz oder teilweise in den Reaktor zurückgefahren werden kann. (Trennung des Destillates und Rückführung eines Teils des organischen Destillats in Figur 2 nicht eingezeichnet) Ein solches Verfahren ist beispielsweise in DE 199 56 410 beschrieben.

### Verfahrensvarianten

An Hand der Figuren 1 und 2 wird die vorliegende Erfindung nachfolgend näher beschrieben.

Ein Blockschema einer Ausführungsform, in der das erfindungsgemäße Verfahren durchgeführt werden kann, ist in Figur 1 dargestellt. Die Einsatzaldehyde als Edukt (1) und die wässrige Base als katalytisch wirkende Prozesslauge (8), bestehend aus Rücklauge (6) und Frischlauge (7), wird von der Reaktionsmischpumpe (RMP) angesaugt. In der Reaktionsmischpumpe (RMP) findet die Aldolkondensation statt. Der Pumpenaustrag (2) wird im Trennbehälter (3) in eine organische Phase (4) mit dem Zielprodukt und eine Laugenphase getrennt, von der ein Teil (5) ausgeschleust und der andere Teil (6) in die Reaktionsmischpumpe (RMP) zurückgeführt wird.

Figur 2 stellt eine weitere Ausführungsform des erfindungsgemäßen Verfahrens dar. Die Verfahrensvariante nach Figur 2 unterscheidet sich von der Variante nach Figur 1 dadurch, dass der Pumpenaustrag (2) vor der Phasentrennung im Behälter (3) einer Kurzdestillation im Apparat (9) unterworfen wird, wobei ein Teil der Leichtsieder (10), hauptsächlich nicht umgesetztes Edukt, abgetrennt wird.

### Beispiele

Die folgenden Beispiele sollen die Erfindung erläutern, ohne die Anwendungsbreite einzuschränken, die sich aus der Beschreibung und den Patentansprüchen ergibt.

### Versuchsapparatur

Die Aldolkondensation von C₅-Aldehyden nach dem erfindungsgemäßen Verfahren erfolgte in einer Versuchsanlage, die schematisch der in Figur 1 dargestellten Verfahrensvariante entsprach.

Die kontinuierliche Katalysatorphase (Rücklauge) 6 und 7 (frische Natronlauge) wird über einen Eintragstutzen in die Reaktionsmischpumpe vom Typ HR060 der Fa. Fink zugeführt und im Kreis geführt. Über einen zweiten Eintragstutzen an der Reaktionspumpe wurde der C₅-Aldehyd (n-Pentanal) durch Leitung 1 zugemischt. Der anfallende Flüssigkeitsstrom nach der Reaktionspumpe (Produkt- und Katalysatorphase) wurde über Leitung 2 in einen Phasentrennbehälter 3 geleitet. Hier wurde die wässrige Katalysatorphase (untere Phase) abgetrennt und über Leitung 6 erneut dem Kreislauf zugeführt. Die organische Phase (obere Phase), die das Reaktionsprodukt enthält, kann über Leitung 4 entnommen werden. Das gebildete Reaktionswasser kann über Leitung 5 kontinuierlich ausgeschleust werden. Um die Verluste an Natronlauge durch Ausschleusung des Reaktionswassers zu kompensieren, wird über die Leitung 7 kontinuierlich frische 10%ige Natronlauge zudosiert. Die Reaktionswärme wurde über die außerhalb des Reaktors liegenden Wärmetauscher (nicht in Figur 1 angezeichnet) abgeführt. Für die Durchführung einer vergleichenden, nicht erfindungsgemäßen Aldolkondensation von Pentanalen wurde, wie im Beispiel I dargestellt, statt einer Reaktionsmischpumpe als Reaktor ein Rührreaktor verwendet.

Die den Beispielen 1 bis 2 beigefügten Tabellen beschreiben im oberen Bereich der Tabellen die Reaktionsbedingungen der C₅-Aldehydkondensation. Im unteren Bereich der Tabelle jedes Beispiels ist die Produktzusammensetzung ebenfalls in Massenprozenten der GC-Analyse aufgelistet. Für eine bessere Übersicht wird nicht zwischen den Isomeren der einzelnen C₁₀-Aldehyden bzw. C₁₀-Hydroxyalkanalen (Aldolen) unterschieden. Diese Werte sind als "2-Propylheptenal" bzw. "C₁₀-Aldol" zusammengefasst. Ebenfalls zusammengefasst als "Hochsieder/Rest" sind die Nebenprodukte der Aldolisierung, wie Trimere und Tetramere, die aus der Aldolreaktion (Addition und Kondensation) von drei bzw. vier C₅-Aldehyden hervorgegangen sind.

### Beispiel 1 (Vergleichsbeispiel):

### Herstellung von 2-Propylheptenal aus n-Pentanal in einem Rührreaktor

2-Propylheptenal wurde durch Kondensation von n-Pentanal in einem Rührreaktor in Form einer Extraktionskolonne (Volumen 2,1 Liter) mit 10 Mischkammern, die mit 4-Blatt-Rührer angebracht auf einer Rührachse ausgestattet waren, hergestellt. Die kontinuierliche Katalysatorphase (2%ige Natronlauge) wurde mittels einer Kreislaufpumpe im Kreis geführt. Das Edukt n-Valeraldehyd wurde aus einem 100 I Fass (Eduktvorlage) entnommen und kontinuierlich über eine dünne Kapillare vor dem Reaktoreintritt in den NaOH-Kreislauf gepumpt. Das Gemisch aus der Produkt- und wässriger Katalysatorphase wurde nach dem Reaktor einem Phasentrennbehälter zugeführt. In dem Phasentrennbehälter wurde die organische Produktphase bei 80 °C von der Katalysatorphase abgetrennt. Nach der Abtrennung der Produktphase wurde die wässrige Phase dem NaOH-Kreislauf zugeführt.

Der Katalysatorkreislauf (2,0%-ige wässrige NaOH) betrug bei allen Versuchen 80 l/h. Das Edukt n-Pentanal wurde mit einem Durchsatz von 8 l/h, entsprechend einem Phasenverhältnis(PV) organische Phase zur wässrige Phase von 1 zu 10, in den NaOH-Kreislauf eingespeist. Das Edukt enthielt neben 99,12 Massen-% n-Pentanal, 0,88 Massen-% Nebenkomponenten, darunter 0,70 Massen-% Hochsieder/Rest. In der folgenden Tabelle 1 sind die Ergebnisse der Aldolisierung von n-Pentanal bei drei Temperaturen 110 °C, 120 °C und 130 °C und 4 bar Druck bei einer Rührdrehzahl von 2000 Upm (Einheit: Umdrehung pro Minute / Upm) dargestellt. Im kontinuierlichen Betrieb nach 3 Stunden Versuchzeit im stationären Zustand wurden folgende Ergebnisse erhalten:

**Tabelle 1. Aldolisierung von n-Pentanal im Rührkessel**

| **Reaktionsbedingungen** | | | |
|---|---|---|---|
| n-Pentanal (l/h) | 8 | 8 | 8 |
| Temperatur (°C) | 110 | 120 | 130 |
| PV (l Edukt/l Kat.-Phase) | 1 zu 10 | 1 zu 10 | 1 zu 10 |

| **Produkt-Zusammensetzung** | | | |
|---|---|---|---|
| n-Pentanal (Massen-%) | 6,33 | 5,34 | 4,43 |
| n-Pentanol (Massen-%) | 0,12 | 0,10 | 0,11 |
| 2-Propylheptenal (Massen-%) | 90,86 | 92,04 | 93,27 |
| C₁₀-Aldole (Massen-%) | 0,93 | 0,58 | 0,00 |
| Hochsieder / Rest (Massen-%) | 1,76 | 1,93 | 2,20 |
| | | | |
| **n-Pentanal-Umsatz (%)** | 93,6 | 94,6 | 95,5 |
| **Selektivität (%)** | 97,9 | 98,1 | 98,5 |

Wie man aus der Tabelle entnehmen kann, steigt der n-Pentanal-Umsatz mit der Reaktionstemperatur an. Für die Erzielung hoher n-Pentanal-Umsätze größer als 95 % werden unter den gewählten Reaktionsbedingungen Reaktionstemperaturen von über 120 °C erforderlich.

### Beispiel 2 (erfindungsgemäß):

### Herstellung von 2-Propylheptenal aus n-Pentanal in einer Reaktionsmischpumpe.

Im folgenden Beispiel wird die Herstellung von 2-Propylheptenal durch Kondensation von n-Pentanal in einer Reaktionsmischpumpe beschrieben. Als Reaktionsmischer wurde Reaktionspumpe vom Typ HR060 mit beheiztem Gehäuse der Fa. Fink mit einem Pumpenhub von rund 15 ml eingesetzt.

Die kontinuierliche Katalysatorphase (2%ige Natronlauge) wurde über einen Eintragstutzen in die Reaktionsmischpumpe geführt. Das Edukt n-Valeraldehyd wurde aus einem 100 I Fass (Eduktvorlage) entnommen und kontinuierlich über einen zweiten Eintragstutzen in das Mischkammer der Reaktionspumpe gepumpt. Das Gemisch aus der Produkt- und wässriger Katalysatorphase wurde aus der Reaktionspumpe über einen Auslassstutzen abgeführt und eine Leitung aus Edelstahl (2 m Länge, 12 mm Innendurchmesser) einem Phasentrennbehälter zugeführt. In dem Phasentrennbehälter wurde die organische Produktphase bei 80 °C von der Katalysatorphase abgetrennt. Nach der Abtrennung der Produktphase wurde die wässrige Phase dem NaOH-Kreislauf zugeführt.

Der Katalysatorkreislauf (2,0%-ige wässrige NaOH) von 160 l/h. wurde bei allen Versuchseinstellungen konstant gehalten. Das Edukt n-Pentanal wurde mit einem Durchsatz von 2 l/h in den Reaktionsmischer eingespeist, entsprechend einem Phasenverhältnis(PV) organische Phase zur wässrige Phase von 1 zu 80. Das Edukt enthielt neben 98,82 Massen-% n-Pentanal, 1,18 Massen-% Nebenkomponente, darunter 0,07 Massen-% n-Pentanol, 0,53 Massen-% 2-Propylheptenal und 0,58 Massen-% Hochsieder/Rest.

In der folgenden Tabelle 2 sind die Ergebnisse der Aldolisierung von n-Pentanal bei drei Reaktionstemperaturen von 110 °C, 120 °C und 130 °C und 5 bar Druck dargestellt.

Im kontinuierlichen Betrieb nach 3 Stunden Versuchzeit im stationären Zustand wurden folgende Ergebnisse erhalten:

**Tabelle 2. Aldolisierung von n-Pentanal in einer Reaktionsmischpumpe**

| **Reaktionsbedingungen** | | | |
|---|---|---|---|
| n-Pentanal (l/h) | 2 | 2 | 2 |
| Temperatur (°C) | 110 | 120 | 130 |
| PV (l Edukt/l Kat.-Phase) | 1 zu 80 | 1 zu 80 | 1 zu 80 |

| **Produkt-Zusammensetzung** | | | |
|---|---|---|---|
| n-Pentanal (Massen-%) | 5,91 | 5,24 | 4,36 |
| n-Pentanol (Massen-%) | 0,10 | 0,10 | 0,11 |
| 2-Propylheptenal (Massen-%) | 89,98 | 91,69 | 93,43 |
| C₁₀-Aldole (Massen-%) | 2,95 | 1,88 | 0,96 |
| Hochsieder / Rest (Massen-%) | 1,05 | 1,09 | 1,15 |
| | | | |
| **n-Pentanal-Umsatz (%)** | 94,0 | 94,7 | 95,6 |
| **Selektivität (%)** | 96,3 | 97,4 | 98,3 |

Wie man aus der Tabelle entnehmen kann, werden bei dem Einsatz einer Reaktionspumpe für die n-Pentanal-Aldolisierung unter den gewählten Reaktionsbedingungen bei Temperaturen oberhalb von 120 °C trotz geringeren Reaktionsvolumens hohe n-Pentanal-Umsätze > 94 % und 2-Propylheptenal-Selektivitäten > 97 % erzielt.

### Beispiel 3 (erfindungsgemäß):

### Herstellung von 2-Propylheptenal aus n-Pentanal in einer Reaktionsmischpumpe

Im folgenden Beispiel wird die Herstellung von 2-Propylheptenal bei einen Pentanal-Durchsatz von rund 1 l/h und einem Phasenverhältnis von 1 zu 160 dargestellt. Im Vergleich zum Beispiel 2 wurde bei sonst gleichen Reaktionsbedingungen der n-Pentanal-Durchsatz halbiert.

Tabelle 3 gibt die Versuchsergebnisse der Aldolisierung bei drei Reaktionstemperaturen von 110 °C, 120 °C und 130 °C und 5 bar Druck wieder. Im kontinuierlichen Betrieb nach 3 Stunden Versuchzeit im stationären Zustand wurden folgende Ergebnisse erhalten:

**Tabelle 3. Aldolisierung von n-Pentanal in einer Reaktionsmischpumpe**

| **Reaktionsbedingungen** | | | |
|---|---|---|---|
| n-Pentanal (l/h) | 1 | 1 | 1 |
| Temperatur (°C) | 110 | 120 | 130 |
| PV (l Edukt/l Kat.-Phase) | 1 zu 160 | 1 zu 160 | 1 zu 160 |

| **Produkt-Zusammensetzung** | | | |
|---|---|---|---|
| n-Pentanal (Massen-%) | 4,05 | 3,03 | 2,77 |
| n-Pentanol (Massen-%) | 0,10 | 0,10 | 0,11 |
| 2-Propylheptenal (Massen-%) | 89,98 | 91,69 | 95,37 |
| C₁₀-Aldole (Massen-%) | 1,37 | 0,57 | 0,35 |
| Hochsieder / Rest (Massen-%) | 1,22 | 1,36 | 1,40 |
| | | | |
| **n-Pentanal-Umsatz (%)** | 95,9 | 96,9 | 97,2 |
| **Selektivität (%)** | 97,8 | 98,6 | 98,7 |

Wie man aus der Tabelle zu entnehmen ist, konnten die n-Pentanal-Umsätze im Vergleich zum Beispiel 2 durch Verringerung des n-Pentanal-Durchsatzes verbessert werden. So wurden bei Reaktionstemperaturen von 120 °C und 130 °C wurden bei Selektivitäten größer als 98 % hohe n-Pentanal-Umsätze von rund 97 % erzielt.

### Beispiel 4 (erfindungsgemäß):

### Herstellung von 2-Propylheptenal aus n-Pentanal in einer Reaktionsmischpumpe

Im folgenden Beispiel wird der Einfluss der Erhöhung des NaOH- Kreislaufes bei konstant gehaltenem Pentanal-Durchsatz auf den Umsatz und Selektivität der Aldolisierung. Hierzu wurde bei 120 °C und 5 bar und einem Pentanal-Durchsatz von 2 l/h der Katalysatorkreislauf zwischen 80 und 240 l/h variiert.

Tabelle 4 gibt die Versuchsergebnisse der Aldolisierung bei konstantem Aldehyd-Durchsatz für drei der Kreislaufeinstellung 80, 160 und 240 l/h wieder. Im kontinuierlichen Betrieb nach drei Stunden Versuchzeit im stationären Zustand wurden folgende Ergebnisse erhalten:

**Tabelle 4. Aldolisierung von n-Pentanal in einer Reaktionsmischpumpe**

| **Reaktionsbedingungen** | | | |
|---|---|---|---|
| n-Pentanal (l/h) | 2 | 2 | 2 |
| Temperatur (°C) | 120 | 120 | 120 |
| NaOH-Kreislauf (l/h) | 80 | 160 | 240 |
| PV (I Edukt/l Kat.-Phase) | 1 zu 40 | 1 zu 80 | 1 zu 120 |

| **Produkt-Zusammensetzung** | | | |
|---|---|---|---|
| n-Pentanal (Massen-%) | 6,56 | 5,24 | 5,22 |
| n-Pentanol Massen-%) | 0,11 | 0,10 | 0,11 |
| 2-Propylheptenal (Massen-%) | 89,39 | 91,69 | 91,94 |
| C₁₀-Aldole (Massen-%) | 2,65 | 1,88 | 1,61 |
| Hochsieder / Rest (Massen-%) | 1,10 | 1,09 | 1,13 |
| | | | |
| **n-Pentanal-Umsatz (%)** | 93,4 | 94,7 | 94,7 |
| **Selektivität (%)** | 96,3 | 97,4 | 97,7 |

Wie man aus der Tabelle zu entnehmen ist, konnte führte die Erhöhung des Kreislaufes von 80 auf 160 und 240 l/h, entsprechend einer Erhöhung des Phasenverhältnisses von 1 zu 40 auf 1 zu 80 und 1 zu 120 zu einer deutlichen Steigerung des n-Pentanal-Umsatzes von 93,4 % auf 97,7 %.

## Patentansprüche

1. Verfahren zur Herstellung von α, β-ungesättigten Aldehyden, bei welchem innerhalb eines kontinuierlich durchströmten Reaktors eine katalytische Aldolkondensation von Einsatzaldehyden in Gegenwart einer wässrigen Base durchgeführt wird,
**dadurch gekennzeichnet,**
**dass** es sich bei dem Reaktor um eine Reaktionsmischpumpe handelt, welche die folgenden Merkmale aufweist:
a) ein fest stehendes Pumpengehäuse,
b) ein im Pumpengehäuse drehbar gelagertes Laufrad, welches eine Vielzahl sich radial ersteckende, zum Umfang des Laufrad hin öffnende Mischkammern aufweist,
c) ein die Mischkammern umschließender Mischkanal, welcher sich innerhalb des Pumpengehäuse entlang des Umfanges des Laufrades erstreckt,
d) mindestens ein Zulauf für Reaktionsedukte und ein Ablauf für Reaktionsprodukte, wobei Zulauf und Ablauf über den Mischkanal fluidisch miteinander verbunden sind,
e) ein Motor, mittels welchem das Laufrad rotierend antreibbar ist.

2. Verfahren nach Anspruch 1, **dadurch gekennzeichnet, dass** bei rotierendem Laufrad die Einsatzaldehyde und die wässrige Base als Reaktionsedukte durch einen gemeinsamen oder getrennte Zuläufe in den Mischkanal gelangen, dass die Aldolkondensation innerhalb des Mischkanals erfolgt, und dass α, β-ungesättigte Aldehyde als Reaktionsprodukte durch den Ablauf den Mischkanal verlassen.

3. Verfahren nach Anspruch 2, **dadurch gekennzeichnet, dass** die Aldolkondensation ausschließlich innerhalb des Mischkanals erfolgt.

4. Verfahren nach Anspruch 2 oder 3, **dadurch gekennzeichnet, dass** das Laufrad mit einer Umfangsgeschwindigkeit rotiert, welche größer ist als die mittlere Fließgeschwindigkeit der Reaktanden entlang des Mischkanals.

5. Verfahren nach einem der Ansprüche 2 bis 4, **dadurch gekennzeichnet, dass** die Temperatur im Mischkanal im Bereich von 50 bis 180 °C gehalten wird.

6. Verfahren nach einem der Ansprüche 2 bis 5, **dadurch gekennzeichnet, dass** Mengenverhältnis von wässriger Base zu Einsatzaldehyden im Zulauf im Bereich von 20 bis 400 liegt.

7. Verfahren nach einem der Ansprüche 2 bis 6, **dadurch gekennzeichnet, dass** die Verweilzeit in der Reaktionsmischpumpe 0,1 bis 2 Sekunden beträgt.

8. Verfahren nach einem der Ansprüche 2 bis 7, **dadurch gekennzeichnet, dass** Pentanale zu Decenalen umgesetzt werden.

9. Verfahren nach Anspruch 8, **dadurch gekennzeichnet, dass** ein C₅-Aldehydgemisch mit einem n-Pentanalgehalt von mindestens 90 Massen-% eingesetzt wird.

10. Verfahren nach Anspruch 8, **dadurch gekennzeichnet, dass** ein C₅-Aldehydgemisch mit einem n-Pentanalgehalt von mindestens 95 Massen-% eingesetzt wird.

11. Verfahren nach Anspruch 8, **dadurch gekennzeichnet, dass** die Temperatur im Mischkanal zwischen 110 °C und 140 °C beträgt.

12. Verfahren nach Anspruch 8, **dadurch gekennzeichnet, dass** Mengenverhältnis von wässriger Base zu Einsatzaldehyden im Zulauf im Bereich von 40 bis 240 liegt.

13. Verfahren nach Anspruch 8, **dadurch gekennzeichnet, dass** die Verweilzeit in der Reaktionsmischpumpe 0,1 bis 1,5 Sekunden beträgt,

14. Verfahren nach Anspruch 8, **dadurch gekennzeichnet, dass** der die Reaktionsmischpumpe über den Ablauf verlassende Pumpenaustrag im Temperaturbereich von 70 bis 120 °C einer Phasentrennung unterzogen wird.

## Claims

1. Process for preparing α, β-unsaturated aldehydes, in which a catalytic aldol condensation of starting aldehydes in the presence of an aqueous base is carried out within a reactor through which continuous flow occurs,
**characterized in that**
the reactor is a reaction mixing pump which has the following features:
a) a fixed pump housing,
b) an impeller which is mounted so as to be able to rotate in the pump housing and has a plurality of radially extending mixing chambers which are open in the direction of the circumference of the impeller,
c) a mixing channel which encloses the mixing chambers and extends within the pump housing along the circumference of the impeller,
d) at least one inlet for reaction starting materials and an outlet for reaction products, with inlet and outlet being connected so as to allow fluid flow by the mixing channel,
e) a motor, by means of which the impeller can be rotated.

2. Process according to Claim 1, **characterized in that** when the impeller is rotating, the starting aldehydes and the aqueous base as reaction starting materials go through a joint inlet or separate inlets into the mixing channel, **in that** the aldol condensation occurs within the mixing channel and **in that** α, β-unsaturated aldehydes as reaction products leave the mixing channel through the outlet.

3. Process according to Claim 2, **characterized in that** the aldol condensation occurs exclusively within the mixing channel.

4. Process according to Claim 2 or 3, **characterized in that** the impeller rotates at a circumferential velocity which is greater than the average flow velocity of the reactants along the mixing channel.

5. Process according to any of Claims 2 to 4, **characterized in that** the temperature in the mixing channel is maintained in the range from 50 to 180°C.

6. Process according to any of Claims 2 to 5, **characterized in that** the ratio of aqueous base to starting aldehydes in the inlet is in the range from 20 to 400.

7. Process according to any of Claims 2 to 6, **characterized in that** the residence time in the reaction mixing pump is from 0.1 to 2 seconds.

8. Process according to any of Claims 2 to 7, **characterized in that** pentanals are converted into decenals.

9. Process according to Claim 8, **characterized in that** a C₅-aldehyde mixture having an n-pentanal content of at least 90% by mass is used.

10. Process according to Claim 8, **characterized in that** a C₅-aldehyde mixture having an n-pentanal content of at least 95% by mass is used.

11. Process according to Claim 8, **characterized in that** the temperature in the mixing channel is in the range from 110°C to 140°C.

12. Process according to Claim 8, **characterized in that** the ratio of aqueous base to starting aldehydes in the inlet is in the range from 40 to 240.

13. Process according to Claim 8, **characterized in that** the residence time in the reaction mixing pump is 0.1 to 1.5 seconds.

14. Process according to Claim 8, **characterized in that** the pump output leaving the reaction mixing pump via the outlet is subjected to a phase separation in the temperature range from 70 to 120°C.

## Revendications

1. Procédé pour la production d'aldéhydes α,β-insaturés, dans lequel on effectue à l'intérieur d'un réacteur traversé en continu une condensation aldolique catalytique d'aldéhydes de départ en présence d'une base aqueuse,
**caractérisée en ce que**
le réacteur consiste en une pompe de mélange-réaction qui présente les caractéristiques suivantes :
a) un corps de pompe fixe,
b) une roue mobile montée de manière à pouvoir tourner dans le corps de pompe, qui comporte une multiplicité de chambres de mélange s'étendant radialement, débouchant sur la périphérie de la roue mobile,
c) un canal de mélange entourant les chambres de mélange, qui s'étend le long de la périphérie de la roue mobile à l'intérieur du corps de pompe,
d) au moins un orifice d'admission pour les produits de départ de la réaction et un orifice d'évacuation pour les produits de la réaction, l'orifice d'admission et l'orifice d'évacuation étant en liaison fluidique entre eux,
e) un moteur, par lequel la roue mobile peut être mise en rotation.

2. Procédé selon la revendication 1, **caractérisé en ce que** lors de la rotation de la roue mobile les aldéhydes de départ et la base aqueuse arrivent en tant que produits de départ de la réaction, par un orifice d'admission commun ou par des orifices d'admission séparés, dans le canal dé mélange, **en ce que** la condensation aldolique s'effectue dans le canal de mélange, et **en ce que** des aldéhydes α,β-insaturés quittent en tant que produits de réaction le canal de mélange par l'orifice d'évacuation.

3. Procédé selon la revendication 2, **caractérisé en ce que** la condensation aldolique s'effectue exclusivement à l'intérieur du canal de mélange.

4. Procédé selon la revendication 2 ou 3, **caractérisé en ce que** la roue mobile tourne à une vitesse périphérique qui est supérieure à la vitesse moyenne d'écoulement des partenaires réactionnels le long du canal de mélange.

5. Procédé selon l'une quelconque des revendications 2 à 4, **caractérisé en ce que** dans le canal de mélange on maintient la température dans la plage de 50 à 180 °C.

6. Procédé selon l'une quelconque des revendications 2 à 5, caractérisé en ce la proportion de la base aqueuse par rapport aux aldéhydes de départ dans l'orifice d'admission se situe dans la plage de 20 à 400.

7. Procédé selon l'une quelconque des revendications 2 à 6, **caractérisé en ce que** le temps de séjour dans la pompe de mélange-réaction va de 0,1 à 2 secondes.

8. Procédé selon l'une quelconque des revendications 2 à 7, **caractérisé en ce qu'**on convertit des pentanals en décénals.

9. Procédé selon la revendication 8, **caractérisé en ce qu'**on utilise un mélange d'aldéhydes en C₅ ayant une teneur en n-pentanal d'au moins 90 % en masse.

10. Procédé selon la revendication 8, **caractérisé en ce qu'**on utilise un mélange d'aldéhydes en C₅ ayant une teneur en n-pentanal d'au moins 95 % en masse.

11. Procédé selon la revendication 8, **caractérisé en ce que** la température dans le canal de mélange est comprise entre 110 °C et 140 °C.

12. Procédé selon la revendication 8, **caractérisé en ce que** la proportion de la base aqueuse par rapport aux aldéhydes de départ se situe dans la plage de 40 à 240.

13. Procédé selon la revendication 8, **caractérisé en ce que** le temps de séjour dans la pompe de mélange-réaction va de 0,1 à 1,5 seconde.

14. Procédé selon la revendication 8, **caractérisé en ce qu'**on soumet à une séparation de phases la décharge de la pompe, quittant par l'orifice d'évacuation la pompe de mélange-réaction, dans une plage de température de 70 à 120 °C.
